# EUROPEAN PATENT SPECIFICATION

(11) **EP 2 800 557 B1**
(45) Date of publication and mention of the grant of the patent: **30.12.2020**
(21) Application number: 13733926.3
(22) Date of filing: 07.01.2013
(51) Int. Cl.: A61K 9/28, A61K 9/00, A61K 9/20, A61K 9/30, A61K 31/465, A61P 25/34, A61P 1/04, A61P 3/04, A61P 25/00, A61P 25/16, A61P 25/28, A61P 1/00

(54) **SOLID NICOTINE-COMPRISING DOSAGE FORM WITH REDUCED ORGANOLEPTIC DISTURBANCE**
FESTE NIKOTINHALTIGE DOSIERUNGSFORM MIT REDUZIERTER ORGANOLEPTISCHER BEEINTRÄCHTIGUNG
FORME POSOLOGIQUE SOLIDE COMPRENANT DE LA NICOTINE À TROUBLES ORGANOLEPTIQUES RÉDUITS

(30) Priority: 05.01.2012 SE 1200017
(43) Date of publication of application: 12.11.2014
(73) Proprietor: McNeil AB, 251 09 Helsingborg (SE)
(72) Inventor: HUGERTH, Andreas, 237 35 Bjärred (SE); LINDELL, Katarina, 241 93 Eslöv (SE); NICKLASSON, Fredrik, 237 33 Bjärred (SE); THYRESSON, Kristina, 226 57 Lund (SE)
(74) Representative: Carpmaels & Ransford LLP
(86) International application number: PCT/SE2013/050005
(87) International publication number: WO 2013/103318

(56) References cited:
- EP-A1- 1 430 896
- EP-A1- 1 666 030
- EP-A1- 2 233 134
- WO-A1-03/003957
- WO-A1-2007/133141
- WO-A1-2009/143841
- WO-A1-2010/044736
- WO-A2-2004/056363
- GB-A- 2 230 439
- US-A- 5 362 496
- US-A1- 2004 151 771
- US-A1- 2011 268 809

## Description

### Technical field

The present invention relates to solid pharmaceutical dosage forms intended for release of nicotine in the oral cavity, such dosage forms being provided with means for reducing one or more organoleptically disturbing sensations.

### Background of the invention

Tobacco dependence and reduction thereof is a desirable goal. In recent years, with the recognition of the harmful effects of tobacco smoking, there have been numerous campaigns and programs by governmental agencies and various health groups and other interested organisations to disseminate information about the adverse health effects resulting from tobacco smoking. Moreover, and as a result of this recognition of the harmful effects, there have been many programs directed to attempts in reducing smoking incidence.

Nicotine is an organic compound and is the principal alkaloid of tobacco. Nicotine is the chief addictive ingredient in the tobacco used in cigarettes, cigars, snuff and the like. Nicotine is also an addictive drug, and smokers characteristically display a strong tendency to relapse after having successfully stopped smoking for a time. Nicotine is the world's second most used drug, after caffeine from coffee and tea.

The main problem with tobacco smoking is its enormous implications on health. According to Centers for Disease Control and Prevention it was estimated that in 2009 smoking related diseases world- wide cause some 5 million deaths per year and that the current trends show that tobacco use will cause more than 8 million deaths annually by 2030. In the United States tobacco use is responsible for one in about five deaths, which means about 450 000 deaths per year. In many large and less developed countries the incidence of tobacco related deaths is even higher. In the United States cigarette smoking costs about 100 billion USD in lost productivity and about 100 billion USD in health care expenditures.

In fact, excessive smoking is now recognised as one of the major health problems throughout the world. This grim consequence of tobacco smoking has urged many medical associations and: health authorities to take very strong actions against the use of tobacco.

Even: though tobacco smoking is decreasing in many developed countries today it is hard to see how the societies could get rid of the world's second most used drug. The incidence of smoking is still rising in many countries, especially in less developed countries.

The most advantageous thing a heavy smoker can do is to stop smoking completely or at least to reduce his/her smoking. Experience shows, however, that most smokers find this extremely difficult since, mostly, tobacco smoking results in a dependence disorder or craving. The World Health Organization ("WHO") has in its International Classification of Disorders a diagnosis called Tobacco Dependence. Others like the American Psychiatric Association call the addiction Nicotine Dependence. It is generally accepted that these difficulties to stop smoking result from the fact that those heavy smokers are dependent on nicotine. The most important risk factors related to health are, however, substances that are formed during the combustion of tobacco, such as carcinogenic far products, carbon monoxide, N-nitrosamines, aldehydes, and hydrocyanic acid.

### Effects of nicotine

Nicotine is an addictive alkaloid, C₅H₄NC₄H₇NCH₃, derived from the tobacco plant. Nicotine is also used as an insecticide. The administration of nicotine (for example, in the form of smoking a cigarette, cigar or pipe) can give a pleasurable feeling to the smoker. However, smoking has health hazards and it is, therefore, desirable to formulate an alternative way of administering nicotine in a pleasurable and harmless manner that can be used to facilitate withdrawal from smoking and/or used as a replacement for smoking.

When smoking a cigarette, nicotine is quickly absorbed into the smoker's blood and reaches the brain within around ten seconds after inhalation. The quick uptake of nicotine gives the consumer a rapid satisfaction, or kick. The satisfaction usually lasts during the smoking time of the cigarette and for a period of time thereafter. The poisonous, toxic, carcinogenic, and addictive nature of smoking has provided strong motivation to develop methods, compositions and devices, which can be used to break the habit of smoking cigarettes.

### Nicotine replacement products

One way to reduce smoking is to provide nicotine in a form or manner other than by smoking and some products have been developed to fulfil: this need. Nicotine containing formulations are currently the dominating treatments for tobacco dependence.

The successes in achieving reduction in the incidence of smoking have been relatively poor using presently known products. The present state of the art involves both behavioural approaches and pharmacological approaches. More than 80 % of the tobacco smokers who initially quit smoking after using some behavioural or pharmacological approach to singly reduce smoking incidence generally relapse and return to the habit of smoking at their former rate of smoking within about a one year's period of time.

As an aid for those who are willing to stop smoking there are several ways and forms of nicotine replacement products available on the market. Several methods and means have been described for diminishing the desire of a subject to use tobacco, which comprises the step of administering to the subject nicotine or a derivative thereof as described in e g U.S. Patent Number 5,810,018 (oral nicotine-containing spray), U.S. Patent Number 5,939,100 (nicotine- containing micro spheres) and U.S. Patent Number 4,967,773 (nicotine-containing lozenge).

Nicotine-containing nose drops have been reported (Russell et al., British Medical Journal, Vol. 286, p. 683 (1983); Jarvis et al., Brit. J. of Addiction, Vol. 82, p. 983 (1987)). Nose drops, however, are difficult to administer and are not convenient for use at work or in other public situations. Administrating nicotine by way of delivering directly into the nasal cavity by spraying is known from U.S. Patent Number 4,579,858, DE 32 41 437 and WO93/12764. There may be local nasal irritation, however, with use of nasal nicotine formulations. The difficulty in administration also results in unpredictability of the dose of nicotine administered.

The use of skin patches for transdermal administration of nicotine has been reported (Rose, in Pharmacologic Treatment of Tobacco Dependence, (1986) pp. 158-166, Harvard Univ. Press). Nicotine-containing skin patches that are in wide use today can cause local irritation and the absorption of nicotine is slow and affected by cutaneous blood flow.

Also, inhaling devices resembling a cigarette are known for uptake of nicotine vapours as suggested in U.S. Patent Number 5,167,242. Said means and methods address the problems associated with addiction to nicotine.

One successful product that is used as a smoking substitute and/or as a smoking cessation aid and which is based on nicotine is the chewing gum Nicorette®. This product was one of the first nicotine replacement forms that was approved: by the Food and Drug Administration (FDA) and is still one of the most used nicotine replacement products. Nicorette® chewing gum has been on the market in about 60 countries for several years. In this chewing gum the nicotine is present in the form of a complex with an insoluble cation-exchanger (polacrilex) that is dispersed in a gum base. The nicotine is slowly released from the gum due to chewing and will reach similar plasma levels as when smoking a cigarette after about 30 minutes depending on the chewing technique, i e slow or active. Patents related to this product are e g U.S. Patent Number 3,877,468, U.S. Patent Number 3,901,248 and U.S. Patent Number 3,845,217.

Pharmaceuticals intended for oral administration are typically provided in solid form as tablets, capsules, pills, lozenges, or gravies. Rapidly dissolving tablets are often employed in the administration of pharmaceuticals where it is impractical to provide a tablet for swallowing whole, for instance with paediatric patients. Several workers in the field have explored rapidly disintegrative tablets, e g U.S. Patent Nos. 6,106,861 and 6,024,981 and PCT Application No. WO 99/47126.

Pharmaceutical tablets for intraoral delivery of nicotine presently available on the market include Commit® Lozenge or NiQuitin® lozenge, a nicotine-containing tablet manufactured by GlaxoSmithKline, and Nicorette Microtab® Sublingual Tablet, a nicotine-containing tablet manufactured by McNeil AB. Many subjects using said tablets experience organoleptically disturbing sensations induced by the nicotine and/or by excipients.

Hence, although release of nicotine in the oral cavity and/or within the pharynx from solid pharmaceutical dosage forms is a convenient means for administration of nicotine sufficient reduction of organoleptically disturbing sensations induced by the nicotine and/or by non-active excipients of the dosage forms remains an unsolved problem.

### Prior art and problems thereof

Ingredients in the above-mentioned pharmaceutical tablets for intraoral delivery of nicotine, which seemingly could have an effect on reducing organoleptically disturbing sensations, comprise one or more flavoring agents and one or more sweeteners. Hence said one or more flavoring agents and said one or more sweeteners do not sufficiently contribute to reducing the organoleptically disturbing sensations related to intraoral delivery from the tablet. One possible reason to why the one or more flavoring agents and the one or more sweeteners do not sufficiently contribute in reducing said organoleptically disturbing sensations may be that the nicotine has to be dissolved in the saliva in order to be absorbed. Once the nicotine is dissolved in saliva the organoleptically disturbing sensations induced by the nicotine cannot be reduced. The same applies for excipients inducing organoleptically disturbing sensations.

The article "Taste Masking of Ondansetron Hydrochloride by Polymer Carrier System and Formulation of Rapid-Disintegrating Tablets, by Shagufta Khan, Prashant Kataria, Premchand Nakhat, and Pramod Yeole, published June 22, 2007 in AAPS PharmSciTech, discloses taste-masking of the bitter taste of the antiemetic drug ondansetron HCL and subsequent formulation of a rapid-disintegrating tablet (RDT) of the taste-masked drug. Such taste-masking, often called microencapsulation, is though unsatisfactory in the present context. This is because the granules are not intended to release the API in the oral cavity upon being disintegrated from the tablet in the mouth. Hence, coating of individual particles or granules according to the above article does not solve the present problem. In order to be effective NRT product nicotine has to be absorbed primarily by the oral mucosa if orally administered

The tobacco industry knows that menthol overrides the harsh taste of tobacco during smoking and alleviates nicotine's irritating effects, synergistically interacts with nicotine, stimulates the trigeminal nerve to elicit a 'liking' response for a tobacco product, and makes low tar, low nicotine tobacco products more acceptable to smokers than corresponding non-mentholated tobacco products. See Menthol's potential effects on nicotine dependence: a tobacco industry perspective", Valerie B Yerger, Tobacco Control 2011; 20(Suppl 2):ii29eii36. doi:10.1136/tc.2010.041970. This publication though does not disclose any use of menthol for reducing one or more organoleptically disturbing sensations in solid pharmaceutical dosage forms that are characterized in that it is provided with at least one film coating for reduction for release of nicotine in the oral cavity. Furthermore, the current invention is related to the surprising effect of the combination of film coating, flavor and/or sweetener in a solid pharmaceutical dosage form for release of nicotine in the oral cavity and is not restricted to the use of menthol.

Hence, there is a need for a convenient and more efficient way to further reduce said organoleptically disturbing sensations. In particular, there is a need for nicotine replacement therapies suitable for use in humans having improved tolerability when administered orally.

EP1430896 (KYUKYU YAKUHIN KOGYO KK) discloses a multi-laminate film where a nicotine-containing layer is laminated against two coating layers. It is not though disclosed that this formulation comprises any component for reduction of an organoleptically disturbing sensation.

WO03003957 (LAVIPHARMA LAB INC) discloses a quick dissolving oral mucosal drug delivery device, comprising a mucosal drug-containing surface-coat-forming inner layer disposed between two moisture barrier coating layers. It is not disclosed that the two moisture barrier coating layers are devoid of the drug.

WO2004056363 (NICONOVUM AB ET AL) discloses a nicotine-containing particulate material, which may be coated with a film-forming polymer. It is not disclosed that this polymer may be devoid of nicotine.

EP1666030 (PFIZER HEALTH AB) discloses nicotine-containing lozenges. It is not disclosed that these lozenges may be provided with a film coating comprising a film-forming polymer, further said film coating being devoid of nicotine and devoid of buffer.

WO2009143841 (FERTIN PHARMA AS) discloses chewing gums provided with a film coating containing liquid flavouring.

EP2233134 (MCNEIL AB) discloses a multi portion intra-oral dosage form containing nicotine.

US2004151771 (GIN JERRY B ET AL) discloses flavoured dosage forms which provide sustained release of a flavouring from a wet matrix of ethylcellulose.

### Definitions

The below definitions apply *mutatis mutandis* on expressions being similar to those being defined below.

The term "Active Pharmaceutical Ingredient (API)", also called Drug Substance, is herein intended to mean a substance or mixture of substances intended to be used in the manufacture of a drug (medicinal) product and that, when used in the production of a drug, becomes an active ingredient of the drug product. Such substances are intended to provide pharmacological activity or other direct effect in the diagnosis, cure, mitigation, treatment, or prevention of disease or to affect the structure and function of the body.

The term "intraoral" is herein intended to mean within the oral cavity.

The term "release" as a verb is herein intended to mean to liberate an API, here nicotine, from its dosage form and to make the API available in dissolved form for subsequent absorption. The term "release" as a noun is to be understood correspondingly.

The term "organoleptically disturbing sensation" is herein intended to mean a sensation perceived as negative in the oral cavity. Non-limiting examples of such sensations are irritation, acridity, taste alteration and taste blocking, feelings of burning, astringing, bitterness and tingling, off tastes such as sour, salty, metallic, soapy, musty, sulphurous, pungent, fatty and foul tastes. Said organoleptically disturbing sensations may be induced by an API, here nicotine, or by non-active excipients. Non-limiting examples of such sensations specifically induced by nicotine are irritation, acridity, feelings of burning, bitterness and tingling, off tastes such as sour, salty, metallic, soapy, fatty and foul tastes. The present application encompasses organoleptically disturbing sensations regardless of their perceived intensity.

The term "organoleptically disturbing substance" is herein intended to mean a substance that may induce an organoleptically disturbing sensation. Organoleptically disturbing substances may encompass APIs, here nicotine, and non-active excipients. Whether a substance induces an organoleptically disturbing sensation or not may be established by methods: known in the art, such as commonly used methods for characterizing organoleptic parameters of food and beverages, such as wine. Non-limiting examples of such methods are e g found in "Sensory Evaluation A practical Handbook", Sarah E. Kemp, Tracey Hollowood and Joanne Hort, Wiley-Blackwell 2011, "Sensory Evaluation Techniques, Fourth Edition, Morten C. Meilgaard, Gail Vance Civille and B. Thomas Carr, CRC Press 2007, and "Sensory Evaluation of Food, Principles and Practices, Second Edition", Harry T. Lawless and Hildegarde Heymann, Springer 2010.

The term "off taste" is herein intended to mean an unpleasant taste or an unpleasant after taste.

The term "encapsulate" is herein intended to mean cover entirely or partly.

The term "core" is herein intended to mean an uncoated solid pharmaceutical dosage form. In other words a core is what you place a coating on to get a coated solid pharmaceutical dosage form. One may also say that a core is encapsulated with a coating to get a coated solid pharmaceutical dosage form.

### Summary of the invention

The present invention is as defined in the appended claims.

The present invention seeks to address the problem of needing to reduce one or more organoleptically disturbing sensations induced by one or more organoleptically disturbing substances being released in the oral cavity from a solid nicotine-comprising pharmaceutical dosage form.

Thus, the invention provides a solid pharmaceutical dosage form for the release of nicotine in the oral cavity comprising a core encapsulated by at least one film coating, wherein the core comprises nicotine and wherein the film coating comprises at least one film-forming polymer and at least one component for reduction of one or more organoleptically disturbing sensations, and where the at least one film coating is devoid of nicotine and devoid of buffer; wherein said dosage form is a lozenge, a sublingual tablet, a buccal tablet, or an orally disintegrating tablet.

Optionally the dosage form may comprise a further API, e g zinc acetate and other salts or complexes with zinc.

Said reduction in organoleptically disturbing sensations should preferably not noticeably deteriorate the pharmaceutical effect of the nicotine or the API.

The invention further provides therapy systems comprising a therapy system of the invention together with one or more further nicotine replacement therapies (such as transdermal patches, gums, mouth sprays, and the like).

The dosage forms and therapy systems of the invention may be used in human medicine in the treatment of a disease selected from the group consisting of tobacco or nicotine dependence, Alzheimer's disease, Crohn's disease, Parkinson's disease, Tourette's syndrome, ulcerous colitis and post-smoking-cessation weight gain.

### Detailed description of the invention

The present solid pharmaceutical dosage form mainly erodes in the mouth whereby nicotine is released and exposed to intraoral sensory receptors, e g taste receptors and trigeminal receptors. Preferably the nicotine is essentially absorbed by the mucosa of the oral cavity. Said pharmaceutical dosage form is a lozenge, a sublingual tablet, a buccal tablet, or an orally disintegrating tablet, which are examples of tablet dosage forms intended to be completely dissolved in the oral cavity. Said solid pharmaceutical dosage form is not intended to be swallowed.

The nicotine is preferably for treating tobacco dependence.

The nicotine may be in any pharmaceutically-acceptable form, such as a nicotine salt, the free base form of nicotine, a nicotine derivative, such as a nicotine cation exchanger, a nicotine inclusion complex or nicotine in any non-covalent binding, nicotine bound to zeolites, nicotine bound to cellulose including micro-crystalline cellulose, or starch micro-spheres and/or mixtures thereof.

The present problem is also of specific interest for certain excipients, non-limiting examples of which are buffers, such as carbonate (including bicarbonate or sesquicarbonate), glycinate, different phosphate systems such as trisodium phosphate, disodium hydrogen phosphate; and tripotassium phosphate, dipotassium hydrogen phosphate, glycerophosphate or citrate of an alkali metal (such as potassium or sodium, or ammonium), e g trisodium and tripotassium citrate, different hydroxides, amino acids, and mixtures thereof, and other excipients that may induce organoleptically disturbing sensations.

When you administer an API, such as nicotine, with a solid pharmaceutical dosage form the API is continuously released as long as the dosage form remains in the mouth. If you do not suck or otherwise mechanically process the dosage form, less API, and less excipients, is released compared to if you suck and/or otherwise mechanically process it. By stopping to suck and/or otherwise mechanically process the dosage form said organoleptically disturbing sensations are normally still not sufficiently reduced.

One way to sufficiently reduce said organoleptically disturbing sensations for a lozenge or a sublingual tablet could be to remove the dosage form from the mouth and put it back into the mouth once the organoleptically disturbing sensations have sufficiently waned. This is though a very inconvenient way to reduce said organoleptically disturbing sensations. For fast dissolving tablets and rapidly disintegrating tablets this option is not available as these tablets would fall apart if they should be taken out from the mouth.

The intention with the: present invention is though to keep the dosage form in the oral cavity until substantially dissolved or disintegrated: and still reduce organoleptically disturbing sensations. If the dosage form instead would be temporarily removed from the mouth as described above this would be not only very inconvenient, but the release of the: API would be temporarily stopped, which normally is unwanted *inter alia* because that may affect the intended dosage regime.

Pharmaceutical tablets for intraoral delivery of nicotine presently available on the market include Commit® Lozenge or NiQuitin® lozenge, a nicotine-containing tablet manufactured by GlaxoSmithKline, and Nicorette Microtab® Sublingual Tablet, a nicotine-containing tablet manufactured by McNeil AB. Many subjects using said tablets experience organoleptically disturbing sensations induced by the nicotine and/or by excipients.

Ingredients in said tablets, which seemingly could have an effect on reducing organoleptically disturbing sensations, comprise one or more flavoring agents and one or more sweeteners. Hence said one or more flavoring agents and said one or more sweeteners do not sufficiently contribute to reducing the organoleptically disturbing sensations related to intraoral delivery from the tablet. One reason to why the one or more flavoring agents and the one or more sweeteners do not sufficiently contribute in reducing said organoleptically disturbing sensations may be that nicotine has to be dissolved in the saliva in order to be absorbed. Once the nicotine is dissolved in the oral cavity the organoleptically disturbing sensations induced by the nicotine cannot be reduced. The same applies for excipients inducing organoleptically disturbing sensations.

The present invention provides a solution to the above-mentioned problem of reducing one or more organoleptically disturbing sensations induced by one or more organoleptically disturbing substances being released in the oral cavity and/or within the pharynx from a solid nicotine-comprising pharmaceutical dosage form. The solution resides in providing said solid dosage form with at least one film coating for reduction of one or more organoleptically disturbing sensations comprising at least one film-forming polymer and at least one component for reduction of one or more organoleptically disturbing sensations, which in combination reduce at least one of said organoleptically disturbing sensations.

Said at least one component for reduction of one or more organoleptically disturbing sensations may by way of example, but not exclusively, be one or more flavoring agents and/or one or more sweeteners.

Said at least one film coating is devoid of nicotine and devoid of buffer. Preferably said at least one film coating is devoid of nicotine and devoid of any other API.

Said reduction of organoleptically disturbing sensations preferably does not significantly affect the release of the nicotine.

The core of the present solid dosage form preferably has a weight from 50 mg to 2000 mg, more preferably from 90 mg to 1200 mg. The film coating on the core preferably has a weight of from 1 % to 15 % of the weight of the core.

The thickness of the film coating has an influence on the degree of reduction of the organoleptically disturbing sensations. Preferably the film coating has an average thickness from 10 to 500 microns, more preferably from 20 to 250 microns, and most preferably from 30 to 150 microns. The actual film thickness is adapted in dependence of different parameters, such as the organoleptic sensation to be reduced, the concentration of flavour, the type of flavour sweetness compounds used and their relative levels and amounts used. The film thickness may be measured using different methods known in the art such as SEM (Scanning Electron Microscopy), digital micrometer, X-ray microtomography, terahertz pulsed imaging etc. See further e g Quantitative Analysis of Film Coating in a Pan Coater Based on In-Line Sensor Measurements, Jose D. Perez-Ramos et al, AAPS PharmSciTech 2005; 6 (1) Article 20, Nondestructive analysis of tablet coating thicknesses using terahertz pulsed imaging. J Pharm Sci. 2005; 94:177Y183. Fitzgerald AJ, Cole BE, Taday PF., Hancock B, Mullarney MP. X-ray microtomography of solid dosage forms. Pharm Technol. 2005;29:92Y100.

A rapid dissolution or disintegration of the at least one film coating is instrumental for not impairing the release of the nicotine. Hence, it is of importance that to an essential degree the at least one film coating dissolves or disintegrates rapidly, preferably in less than 2 minutes, more preferably in less than 1 minute and most preferably in less than 30 seconds, from the moment of administration

Too long a time for release of the nicotine may impair the user friendliness. Hence, the solid dosage form may preferably release the nicotine within 30 minutes, more preferably within 15 minutes, from the moment of administration.

The film-forming polymers may in a non-limiting way be chosen among cellulose ethers e g hydroxy propyl methyl cellulose (HPMC), methyl hydroxy ethyl cellulose (MHEC), hydroxy propyl cellulose (HPC), hydroxyethyl cellulose (HEC), ethyl hydroxyl ethyl cellulose (EHEC), and other film forming polymers such as methacrylic acid copolymer-type C, sodium carboxy methyl cellulose, polydextrose, polyethylene glycols, acrylate polymers (e g poly vinyl acrylate (PVA)),polyvinyl alcohol-polyethylene glycol graft copolymers, complex of polyvinylpyrrolidone (PVP), such as povidone, polyvinyl alcohol, microcrystalline cellulose, carrageenan, pregelatinized starch, polyethylene glycol, and combinations thereof. Typically, the molecular weight (weight average and/or number average) of the polymer is from 1,000 to 10,000,000, preferably from 10,000 to 1,000,000, as measured by gel permeation chromatography.

Optionally, a plasticizer may be added to the film-forming polymer to facilitate the spreading and film forming capability. Examples on useful plasticizers are glycerol, propylene glycol, polyethylene glycol (PEG 200-6000), organic esters e g triacetin (glyceryl triacetate), triethyl citrate, diethyl phtalate, dibutyl phtalate, dibutyl sebacete, acetyltriethyl citrate, acethyltributyl citrate, tributyl citrate, and oils/glycerides such as fractionated coconut oil, castor oil and distilled acetylated monoglycerides. Additionally, or alternatively, surfactants may be included to facilitate the incorporation of flavors and to improve penetration and spreading properties of the coating liquid. Non-limiting examples of surfactant are polysorbates derived from PEG-ylated sorbitan esterified with fatty :acids such as Polysorbate 20 (Polyoxyethylene (20) sorbitan monolaurate), Polysorbate 40 (Polyoxyethylene (20) sorbitan monopalmitate), Polysorbate 60 (Polyoxyethylene (20) sorbitan monostearate), Polysorbate 80 (Polyoxyethylene (20) sorbitan monooleate) (e g Tween 80, Tween 40, Tween 20), sodium lauryl sulphate (SLS), poloxamer surfactants i.e. surfactants based on ethylene oxide - propylene oxide block copolymers and other surfactants with high HLB-value.

Anti-tacking agents/glidants may in a non-limiting way be chosen among compounds such as talc, magnesium stearate, kaolin, colloidal silicon dioxide and glyceryl monostearate. The aforementioned agents may also be included to reduce sticking issues.

The flavoring agents may in a non-limiting way be chosen among natural or synthetic flavouring or aromatizing agents and may be added as liquids and/or as powder. Flavour and aroma agents may be selected from essential oils including distillations, solvent extractions, or cold expressions of chopped flowers, leaves, peel or pulped whole fruit comprising mixtures of alcohols, esters, aldehydes and lactones; essences including either diluted solutions of essential oils, or mixtures of synthetic chemicals blended to match the natural flavour of the fruit, (e g strawberry, raspberry, black currant, banana, melon, cherry, passion fruit, pineapple, peach, blackberry, mango, papaya, guava, cranberry, cloudberry, violet, pomegranate, pear, apple); artificial and natural flavours of brews and liquors, (e g cognac, whisky, rum, gin, sherry, port, and wine); tobacco, coffee, tea, cocoa, and mint; fruit juices including expelled juice from washed, scrubbed fruits such as lemon, orange, lime and other citric fruits; spear mint, pepper mint, lemon balm, wintergreen, cinnamon, cacoe/cocoa, vanilla, liquorice, menthol, eucalyptus, aniseeds, nuts (e g peanuts, coconuts, hazelnuts, chestnuts, walnuts, cobnuts), almonds, raisins and ginger; and powder and flour.

The sweeteners may in a non-limiting way be chosen among synthetic or natural sugars, i e any form of carbohydrates suitable for use as sweetener, as well as so called artificial sweeteners such as saccharin, sodium saccharin, aspartame, e g NutraSweet®, acesulfame or Acesulfame K®, potassium acesulfame, thaumatin, glycyrrhizin, sucralose, dihydrochalcone, miraculin, monellin, stevside, e g Stevia®, neotame, N-substituted APM derivatives, cyclamic acid and its salts and alitame. Sweeteners may also be selected from the group consisting of sugar alcohols, such as sorbitol, xylitol, single sugars including sugars extracted from sugar cane and sugar beet (sucrose), dextrose (also called glucose), fructose (also called leavulose), and lactose (also called milk sugar); sorbitol,: mannitol, glycerol, xylitol, erythritol, maltitol syrup (or hydrogenated starch hydrolyzate), isomalt, lactitol; and mixtures of sugars including glucose syrup, (e g starch hydrolysates, containing a mixture of dextrose, maltose and a range of complex sugars), invert sugar syrup, (e g sucrose inverted by invertase (also called sucrase or sacchrase) containing a mixture of dextrose and fructose), high sugar content syrups such as treacle and honey containing a mixture of particular leavulose, dextrose, maltose, lactitole, sucrose, resins, dextrin and higher sugars; and malt or malt extracts.

Other adjuvants may also be included in the composition of the film such as coloring agents, opacifiers, glossing agents, pore forming agents, excipient stabilizers.

The dosage forms of the invention may be prepared by way of a variety of routine techniques, and using standard equipment, known to the skilled person (see, for example, Lachman et al, "The Theory and Practice of Industrial Pharmacy", Lea & Febiger, 3rd edition (1986) and "Remington: The Science and Practise of Pharmacy", Gennaro (ed.), Philadelphia College of Pharmacy & Sciences, 19th edition (1995)). In one embodiment, a core comprising nicotine is first produced using known tabletting techniques, which is then coated with a solution containing a film-forming polymer.

Standard mixing equipment may be used for mixing together components of compositions of the invention. The mixing time period is likely to vary according to the equipment used, and the skilled person will have no difficulty in determining by routine experimentation a suitable mixing time for a given combination of ingredient(s).

Surprisingly, after that the film coating essentially has disappeared from the surface of the solid dosage form the reduction of organoleptically disturbing sensations remains.

Equally surprisingly, when incorporating said components for reducing organoleptically disturbing sensations in the core of the: solid dosage form, instead of incorporating those in the film coating said organoleptically disturbing sensations will not be sufficiently reduced.

Upon it having been dissolved a film coating on its own has a limited effect on the reduction of organoleptically disturbing sensations. A component for reduction of said sensations, such as a flavoring agent or a sweetener, may have a limited effect on its own on the reduction of organoleptically disturbing sensations. Surprisingly the combined effect of a film coating and at least one further component for reduction of said sensations, provides an effect that is more profound than the sum of the effects of the film coating on its own and the at least one further component on its own.

Reducing organoleptically disturbing sensations implies increased therapy adherence, which may lead to increased efficacy of the treatment.

### Examples

The below examples on embodiments and manufacturing of the present formulation as well as on testing the present formulations are non-limiting and for illustrating the present invention. Alternatives and variations of the below examples within the scope of the present invention as per the below claims may be carried out by a person skilled in the art. Ingredients as per the below examples may be exchanged for equivalent ingredients. The combination of tablet cores and film coatings in the examples given are arbitrary. Any film coating can be combined with any tablet core.

### Example 1

### Manufacturing method

The composition for a batch of tablet cores is given below in Table A1. The materials are sieved using an oscillating sieve with 1mm mesh size and thereafter blended, according to methods known in the art e g using a double cone blender for 10 to 30 minutes. The blended materials are then compressed into tablets by means of direct compression. The powder compression may for example be performed using a rotary tablet press with 15 mm round concave punches. The tablets are compressed to sufficient hardness to enable an acceptable coating process and to achieve the desired *in vivo* dissolution time.

**Table 1A: Components of the tablet core.**

| Ingredients | Percent (w/w) | mg/portion |
|---|---|---|
| Nicotine resin complex (20% nicotine) | 1.5 | 15* |
| Sorbitol | 89.0 | 890 |
| Xylitol | 5.75 | 57.5 |
| Sodium bicarbonate | 0.25 | 2.5 |
| Sodium carbonate | 0.5 | 5 |
| Mint flavor | | 10 |
| Magnesium stearate | 1.5 | 15 |
| TOTAL | 100.0 | 1000.0 |

| | | |
|---|---|---|
| * Equivalent to 3.0 mg dose of nicotine base. | | |

Table 1B provides numerous alternative non-limiting examples of tablet core compositions.

**Table 1B: Components of the tablet core.**

| Ingredients | Percent (w/w) | mg/portion |
|---|---|---|
| Nicotine resin complex (20% nicotine)* | 0.25 - 6.0 | 2.5 - 60 |
| Sorbitol | 0 - 99.15 | 0- 991.5 |
| Xylitol | 0 - 99.15 | 0 - 991.5 |
| Mannitol | 0 - 99.15 | 0 - 991.5 |
| Sodium bicarbonate | 0 - 1.0 | 0 - 15 |
| Sodium carbonate | 0 - 1.0 | 0- 15 |
| Flavor (mint and/or fruit and/or other) | 0.05 - 2.5 | 0.5 - 25 |
| Sucralose ** | 0 - 0.25 | 0 - 2.5 |
| Magnesium stearate | 0.5-2.5 | 5-25 |
| TOTAL | 100.0 | 1000.0 |

| | | |
|---|---|---|
| * or other source equivalent to 0.5 mg to 12 mg nicotine base. ** or other high intensity sweetener or combination of such sweeteners. | | |

Film coating of the tablets can be performed using e g a standard modern pan coater equipped inter alia with air atomized spray nozzles to distribute the film coating fluid and a perforated drum of appropriate size. The film solution is prepared by adding the hydroxypropyl methylcellulose and plasticizer (if such is included in the composition) to purified water (>85°C) whilst stirring. The most suitable temperature of the solvent used for dispersing the hydroxypropyl methylcellulose depends on the type of hydroxypropyl methylcellulose used. There is abundant information in the literature regarding hydroxypropyl methylcellulose film preparation e g from polymer manufacturers such as Dow Inc. http://dowwolff.custhelp.com/app/answers/detail/a_id/1094/kw/prepare/session/L3RpbW UvMTMyMzY3MzM3Ny9zaWQvMkFoOUVuTGs%3D and http://dowwolff.custhelp.com/app/answers/detail/aid/1181. The film solution is cooled to approximately 20°C and sucralose is added when the solution is approximately 40°C. The solution is allowed to settle at ambient conditions for at least 3 hours where after the solution is homogenized using a Silverson homogenisator. Thereafter flavor mixture is added containing Polyoxyethylene (80) sorbitan monooleate and mint flavor. The resulting mixture is stirred until it is homogenous. The components of the film coating composition are given below and in other examples are provided as the calculated amount per unit dosage form. The sum of the "dry excipients", also referred to as "solids content" is usually in the range 5-25 % w/v of the total coating solution. The actual solids content chosen depends on the composition and coating process parameters.

**Table 1C: Components of the film coating.**

| Ingredients | Percent (w/w) | mg/portion |
|---|---|---|
| Hydroxypropyl methylcellulose | 79.7 | 19.925 |
| Polyoxyethylene (20) sorbitan monooleate | 0.3 | 0.075 |
| Sucralose* | 8.0 | 2 |
| Mint flavor (or e g Fruit flavor) | 12.0 | 3 |
| Sum "Dry" Excipients | 100.0 | 25 |
| Aqua pur** | q.s. | - |

| | | |
|---|---|---|
| * or other high intensity sweetener or combination of such sweeteners. ** Aqua. Pur. is added q.s. to achieve a dry content suitable for the coating process parameter setting to be applied e.g. dry content in the range 10% w/w to 25% w/w. | | |

A coloring component may also be included, e g titanium dioxide.

**Table 1D: Components of an alternative film coating**

| | Percent (w/w) : | mg/portion |
|---|---|---|
| Hydroxypropyl methylcellulose | 80.45 | 32.28 |
| Polyethyleneqlvcol 400* | 8.2 | 3.28 |
| Polyoxyethylene (20) sorbitan monooleate | 0.1 | 0.04 |
| Titanium dioxide | 6.0 | 2.4 |
| Aspartame | 4.0 | 1.6 |
| Fruit flavor (or eg a Mint flavor) | 1.25 | 0.5 |
| Sum "Dry" Excipients | 100.0 | 40 |
| Aqua pur* | q.s. | |

| | | |
|---|---|---|
| *Aqua. Pur.is added q.s. to achieve a dry content suitable for the coating process parameter setting to be applied. | | |

**Table 1E: Components of additionally non-limiting alternative film coatings**

| Ingredients | Percent (w/w) | mg/portion |
|---|---|---|
| Hydroxypropyl | 44.5-97.0 | 8.9-19.4 |
| Polyethyleneglycol 400² 400² | 0-25 | 0-5 |
| Polyoxyethylene (20) sorbitan monooleate³ | 0-0.5 | 0-0.1 |
| Titanium dioxide (optional ingredient) | 0-10 | 0-2 |
| Sucralose⁴ | 0.5-10 | 0.1-2 |
| Fruit flavor (or e ga Mint flavor)⁵ | 2.5-10 | 0.5-2 |
| Sum "Dry" Excipients | 100.0 | 20 |
| Aqua pur⁶ | q.s. | |

| | | |
|---|---|---|
| ¹ The hydroxypropyl methylcellulose may e g be of type methocel E3, K4, E5 or F_VLV. The hydroxypropyl methylcellulose may also be replaced in part or in its entire by a combination of other film forming polymers. ² May be exchanged for propylene glycol, glycerol triacetin or other plasticizer. ³ May be exchanged for other surfactant. ⁴ Alternatively sodium lauryl sulphate or equivalent surfactant ⁵ Alternatively other high intensity sweetener or combination of such sweeteners. Sweetener may also be included in the flavor. ⁶ Aqua. Pur. is added q.s. to achieve a dry content suitable for the coating process parameter setting to be applied and is essentially evaporated during the process. | | |

### Example 2

Manufacturing method of tablets as per Example 1

**Table 2A: Components of the tablet core,**

| Ingredients | Percent (w/w) | mg/portion |
|---|---|---|
| Nicotine resin (20% nicotine)* | 1.67 | 10.0 |
| Isomalt | 93.56 | 561.36 |
| Sodium carbonate | 0.5 | 3.0 |
| Mint flavor | 1.67 | 10.02 |
| Cooling agent | 0.1 | 0.6 |
| Magnesium stearate | 2 | |
| Silicon dioxide (colloidal) | 0.5 | 3.0 |
| TOTAL | 100 | 600 |

| | | |
|---|---|---|
| * Equivalent to 2.0 mg dose of nicotine base. If nicotine resin complex with other degree of nicotine loading is used, e g 15%, then the amount of polyol is adjusted accordingly. | | |

Film coating of the tablets produced in 2A can be performed using e g a standard modern pan coater equipped with air atomized spray nozzles to distribute the film coating fluid and a perforated drum of appropriate size. The film solution is prepared by adding the hydroxypropyl methylcellulose to aqua purificata during stirring and then the solution is allowed to settle overnight at ambient conditions where after polyvinyl alcohol, polyethylene glycol 400 and sucralose are added during stirring. The solution is homogenized using a Silverson homogenisator. Thereafter flavor mixture containing Polyoxyethylene (80) sorbitan monooleate and mint flavor is added. The resulting mixture is stirred until it is homogenous.

**Table 2B: Components of the film coating.**

| Ingredients | Percent(w/w) | mg/portion |
|---|---|---|
| Hydroxypropyl methylcellulose | 56.5 | 14.13 |
| Polyvinyl alcohol | 12 | 3.0 |
| | 16 | |
| (80) sorbitan monooleate | 0.3 | 0.08 |
| Sucralose | 7.2 | 1.8 |
| Mint flavor | 8 | 2 |
| Sum "Dry" Exipients** | 100 | 20 |
| Aqua pur*** | q.s. | |

| | | |
|---|---|---|
| * Or other plasticizer e g triacetin, i.e. 1,2,3-triacetoxypropane, glycerol or propylenglycol, which usually are used at concentration of 10-35% based on polymer weight. ** Sum excipients other than Aqua pur. ***Aqua. Pur. is added q.s. to achieve a dry content suitable for the coating process parameter setting to be applied e g may the dry content be 16% w/w. | | |

### Example 3

As per Example 2 with a total weight of the tablet core of 650 mg using oval 14.5 mm punches, but without sodium hydrogen carbonate and/or sodium carbonate (which is compensated by amount of Mannitol). Additionally the components of the film coating are provided in Table 3A.

**Table 2B: Components of the film coating.**

| Ingredients | Percent(w/w) | mg/portion |
|---|---|---|
| Hydroxypropyl methylcellulose | 56.5 | 14.13 |
| Polyvinyl alcohol | 12 | 3.0 |
| Polyethyleneglycol 400 | 16.3 | 4.08 |
| Sucralose | 7.2 | 1.8 |
| Mint flavor | 8 | 2 |
| Sum "Dry" Excipients* | 100 | 25.0 |
| Aqua pur** | q.s. | |

| | | |
|---|---|---|
| *Sum excipients other than Aqua pur. **Aqua. Pur. is added q.s. to achieve a dry content suitable for the coating process parameter setting to be applied e g may the dry content be 16% w/w. | | |

### Example 4

Manufacturing method as per Example 1.

**Table 4A: Components of the tablet core.**

| Ingredients | Percent (w/w) | mg/portion |
|---|---|---|
| Nicotine resin complex (20% nicotine)* | 2.0 | 20 |
| Mannitol | 93.5 | 744 |
| Sodium hydrogen carbonate | 0.5 | 4.0 |
| Sodium carbonate | 0.5 | 4.0 |
| Fruit flavor | 1 | 8.0 |
| stearate Magnesium stearate | 2.5 | 20.0 |
| TOTAL | 100 | |

| | | |
|---|---|---|
| * Equivalent to 4.0 mg dose of nicotine base. If equivalent amount of nicotine base is supplied by means of nicotine resin complex with other degree of nicotine loading is used e g 15% or nicotine bitartrate then the amount of polyol is adjusted accordingly. | | |

Film coating of the tablets can be performed using e g a standard modern pan coater equipped inter alia with air atomized spray nozzles to distribute the film coating fluid and a perforated drum of appropriate size.

**Table 4B: Components of the film coating.**

| Ingredients | Percent( w/w) | mg/portion |
|---|---|---|
| Hydroxypropyl methylcellulose | 75 | 26.25 |
| Triacetin* i e 1,2,3-triacetoxypropane | 7.5 | 2.625 |
| Polyoxyethylene (80) sorbitan monooleate | 0.1 | 0.035 |
| Sucralose | 7.4 | 2.59 |
| Mint flavor | 10 | 3.5 |
| Sum "Dry" Exipients | 100 | 35 |
| Aqua pur** | q.s. | |

| | | |
|---|---|---|
| * May be exchanged for another plasticizer, such as polyethyleneglycol 1000, **Aqua. Pur. is added q.s. to achieve a dry content suitable for the coating process parameter setting to be applied. The concentration of hydroxypropyl methylcellulose may for example be 7% w/w. | | |

### Example 5.

Manufacturing method as per Example 1.

**Table 5A: Components of the tablet core.**

| Ingredients | Percent (w/w) | mg/portion |
|---|---|---|
| Nicotine ß-cyclodextrin | 8.55 | 8.55* |
| ß-cyclodextrin | 68.1 | 68.1 |
| Xylitol | 10 | 10 |
| Crospovidone (polyvinylpyrrolidone) | 2 | 2 |
| Sodium carbonate anhydrous | 5 | 5 |
| Mint flavor | 5 | 5 |
| Magnesium stearate | 0.9 | 0.9 |
| Colloidal silicon dioxide | 0.45 | 0.45 |
| TOTAL | 100 | 100 |

| | | |
|---|---|---|
| * Equivalent to 1.0 mg dose of nicotine base. | | |

Film coating of the tablets can be performed using e g a standard modern pan coater equipped inter alia with air atomized spray nozzles to distribute the film coating fluid and a perforated drum of appropriate size.

**Table 5B: Components of the film coating.**

| Ingredients | Percent (w/w) | mg/portion |
|---|---|---|
| Hydroxypropyl methylcellulose | 74 | 7.4 |
| Polyoxyethylene (80) sorbitan monooleate | 0.1 | 0.01 |
| Sucralose | 4.9 | 0.49 |
| Acesulfame potassium (Potassium 6-methyl-2,2-dioxo-oxathiazin-4) | 4 | 0.4 |
| Aspartame ((*N*-(L-α-Aspartyl)-L-phanylalanine) | 2 | 0.2 |
| Mint flavor | 15 | 1.5 |
| Sum "Dry" Exipients | 100 | 10 |
| Aqua pur* | q.s | - |

| | | |
|---|---|---|
| *Aqua. Pur. is added q.s. to achieve a dry content suitable for the coating process parameter setting to be applied, e g 24% w/w. | | |

### Example 6

Manufacturing method as per Example 1.

Components of the tablet core as per Example 4.

Film coating of the tablets can be performed using e g a standard modern pan coater equipped inter alia with air atomized spray nozzles to distribute the film coating fluid and a perforated drum of appropriate size. The film solution is prepared by adding the hydroxypropyl methylcellulose to aqua purificata whilst stirring. The film solution is cooled to approximately 20°C and sucralose and acesulfame K is added when the solution is approximately 40°C. The solution is allowed to settle at ambient conditions for at least 3 hours where after the solution is homogenized using a Silverson homogenisator, Thereafter flavor mixture is added containing Polyoxyethylene (80) sorbitan monooleate and mint flavor. The resulting mixture is stirred until it is homogenous.

**Table 6B: Components of the film coating.**

| Ingredients | Percent (w/w) | mg/portion |
|---|---|---|
| methylcellulose | 70 | 24.3 |
| Titan dioxide | 3 | 1.05 |
| Propylene glycol | 9 | 3.15 |
| Polyoxyethylene (80) sorbitan monooleate | 0.1 | 0.035 |
| Aspartame | 4.9 | 1.715 |
| Acesulfame Potassium | 3 | 1.05 |
| Mint flavor | 10 | 3.5 |
| Sum "Dry" Exipients | 100 | 35 |
| Aqua pur* | q.s | - |

| | | |
|---|---|---|
| * Aqua. Pur. is added q.s. to achieve a dry content suitable for the coating process parameter setting to be applied. | | |

### Example 7

As per Example 6, but with the following film coating composition

**Table 7B: Components of the film coating.**

| Ingredients | Percent (w/w) | mg/portion |
|---|---|---|
| Hydroxypropyl methylcellulose | 77.3 | 11.595 |
| Titan dioxide | 3 | 0.45 |
| Polyethyleneglycol 400 | 1.5 | 0.225 |
| Sodium lauryl sulfate | 0.3 | 0.045 |
| Aspartame | 4.9 | 0.735 |
| Acesulfame Potassium | 3 | 0.45 |
| | 10 | 1.5 |
| Sum "Dry" Expients | 100 | 15 |
| Aqua pur* | q.s. | - |

| | | |
|---|---|---|
| * Aqua. Pur. is added q.s. to achieve a dry content suitable for the coating process parameter setting to be applied. | | |

### Example 8

Manufacturing method as per Example 1.
Coating as per Example 4.

**Table 8A: Components of the tablet core.**

| Ingredients | Percent (w/w) | mg/portion |
|---|---|---|
| Nicotine resin complex (20% nicotine)* | 1.25 | 7.5 |
| Mannitol | 93.98 | 563.88 |
| Zinc Acetate dihydrate** | 0.67 | 4.02 |
| Sodium carbonate | 0.5 | 3 |
| Mint flavor | 1 | 6 |
| Cooling agent | 0.1 | 0.6 |
| Magnesium stearate | 2.5 | 15 |
| TOT AL | 100 | 600 |

| | | |
|---|---|---|
| * Equivalent to 4.0 mg dose of nicotine base. If nicotine resin complex with other degree of nicotine loading is used e g 15% then the amount of polyol is adjusted accordingly. ** Equivalent to 2.0 mg dose of zinc. | | |

### Example 9

Manufacturing method of tablet core as per Example 1, but with 6 mm round punches.

**Table 9A: Components of the tablet core,**

| Ingredients | Percent (w/w) | mg/portion |
|---|---|---|
| Nicotine resin complex (20% nicotine)* | 2.5 | 2.5 |
| Mannitol | 72.25 | 72.25 |
| 2-Pyrrolidinone, 1-Etheny Homopolymer (PVP) | 18 | 18 |
| Sodium carbonate anhydrous | 2.5 | 2.5 |
| Mint flavor | 3 | 3 |
| Manesium stearate | 1.75 | 175 |
| TOTAL | 100 | 100 |

| | | |
|---|---|---|
| * Equivalent to 1.5 mg dose of nicotine base | | |

Coating manufacturing process as per Example 1C.

**Table 9B: Components of the film coating.**

| Ingredients | Percent (w/w) | mg/portion |
|---|---|---|
| methylcellulose | 78.8 | 7.68 |
| Polyoxyethylene (80) sorbitan monooleate | 0.2 | 0.02 |
| Aspartame | 4 | 0.4 |
| Acesulfame potassium | 4 | 0.4 |
| Fruit flavor | 15 | 1.5 |
| Sum "Dr" Exipients | 100 | 10 |
| Aqua pur* | q.s. | - |

| | | |
|---|---|---|
| * Aqua. Pur. is added q.s. to achieve a dry content suitable for the coating process parameter setting to be applied, | | |

A coloring component may also be included, e g titanium dioxide,

### Example 10

### Manufacturing method

The compositions for two tablet cores are given below in Table 10A. The master granule materials are sieved using an oscillating sieve with 1mm mesh size and thereafter blended, according to methods known in the art e.g. using a double cone blender for 10 to 30 minutes. The blended materials are then wetted with purified water. The wet mass is then fed to an extruder to form the granules. The resultant granules are dried using any method known in the art, such as fluid bed drying. The master granules are then screened for a suitable particle size, typically 75 µm, 200 mesh. The master granules are then blended with the nicotine active, at least one buffering agent, flavorants and sweeteners. Upon mixing and screening a lubricant or glidant is added to the mixture. The tablets are compressed to sufficient hardness to enable an acceptable coating process and to achieve the desired *in vivo* dissolution time.

**Table 10 A Components of core.**

| Ingredients | Formulation 10A mg/portion | Formulation 10B mg/portion |
|---|---|---|
| *Master granule:* | | |
| Mannitol | 178.8 | 1034.9 |
| Potassium hydrogencarbonate | 0.45 | |
| Sodium carbonate | 3.67 | 22.75 |
| Sodium alginate | 10.30 | 63.70 |
| Xanthan gum | 1.99 | 12.25 |
| Calcium polycarbophil | 5.13 | 31.73 |
| *Dry mixed components.* | | |
| Nicotine resin complex | 22.22 | 22.22 |
| Potassium hydrogencarbonate | 0.58 | |
| Sodium carbonate anhydrous | 4.63 | |
| | | 6.00 |
| Aspartame Acesulfame Potassium | 1.50 | |
| Mint flavor | 21.25 | 1.2 |
| Magnesium stearate | 2.50 | 2.50 |
| Total weight of tablet core mg | 250 | 1200 |

**Table 10 B: Components of film coating,**

| Ingredients | Percent (w/w) | Formulation 10 A mg/portion | Formulation 10 B mg/portion |
|---|---|---|---|
| Hydroxypropyl methylcellulose | 77.3 | 4.83 | 23.19 |
| Titan dioxide | 3 | 0.19 | 0.90 |
| Polyethyleneglycol 400 | 1.5 | 0.094 | 0.45 |
| Sodium lauryl sulfate | 0.3 | 0.019 | 0.09 |
| Aspartame | 4.9 | 0.31 | 1.41 |
| Acesulfame Potassium | | 0.19 | 0.90 |
| Mint flavor | 10 | 3 | 3.00 |
| Sum "Dry" Exipients | 100 | 8.633 | 30 |
| Aqua Pur* | q.s. | | |

| | | | |
|---|---|---|---|
| * Aqua. Pur. is added q.s. to achieve a dry content suitable for the coating process parameter setting to be applied. | | | |

The respective amounts in the two above formulations 10 A and 10 B may vary within an interval of +- 15 % (w/w), preferably within + - 5 % (w/w) without thereby deviating from the desired characteristics for the respective formulations.

### Example 11

Results from a sensory study confirmed the surprising finding of reduction of disturbing sensations. 16 study persons (healthy volunteers; 8 mates and 8 females in age range 34 to 64 years, either smokers or NRT-users) completed the study and compared two nicotine lozenge 4 mg formulations; lozenge A, uncoated, with all of flavoring agents and sweeteners in the tablet core, lozenge B with an additional film coating. The additional film coating for lozenge B carried a portion of flavoring agents and sweeteners, while corresponding amount was withdrawn from the lozenge core. Thus the total amount of flavoring agents and sweeteners was the same in both lozenges. The lozenge cores for both A and B had the same composition except for the amounts of flavoring and sweetening agents.

The result showed that rating of tingling/burning sensation differed to a great extent: 12 out of 16 participants gave the lowest score (almost no burning/tingling) on a five grade scale after 30 seconds of testing for formulation B while only 7 out: of 16 participants gave the lowest score for formulation A. The effect of the film was persistent; after the lozenge had completely dissolved 10 of 16 participants gave the lowest score for formulation B while 7 out of 16 gave the lowest score for formulation A. All study persons tested both formulations with at least 30 minutes between the tests. The scale used for tingling/ burning was a 5-point intensity scale.

## Claims

1. A solid pharmaceutical dosage form for the release of nicotine in the oral cavity comprising a core encapsulated by at least one film coating, wherein the core comprises nicotine and wherein the film coating comprises at least one film-forming polymer and at least one component for reduction of one or more organoleptically disturbing sensations, and where the at least one film coating is devoid of nicotine and devoid of buffer;
wherein said dosage form is a lozenge, a sublingual tablet, a buccal tablet, or an orally disintegrating tablet.

2. Dosage form according to claim 1, **characterized in that** it comprises at least one further active pharmaceutical ingredient, such as zinc salt or zinc complex.

3. Dosage form according to claim 1 or 2, **characterized in that** upon administration said nicotine is essentially absorbable by the mucosa of the oral cavity and/or by the mucosa of the pharynx.

4. Dosage form according to any one of claims 1 to 3, **characterized in that**
the at least one film coating preferably has a thickness from 10 to 500 microns, more preferably from 20 to 250 microns, and most preferably from 30 to 150 microns and/or,
the core preferably has a weight from 50 mg to 2000 mg, more preferably from 90 mg to 1200 mg and/or
the film coating preferably has a weight of from 1 % to 15 % of the weight of the core.

5. Dosage form according to any one of claims 1 to 4, **characterized in that** the organoleptically disturbing sensation/s is/are selected from the group consisting of irritation, acridity, taste alteration and taste blocking, feelings of burning, astringing, bitterness and tingling, and off tastes such as sour, salty, metallic, soapy, musty, sulphurous, pungent, fatty and foul tastes, preferably irritation, acridity, feelings of burning, bitterness and tingling, off tastes such as sour, salty, metallic, soapy, fatty and foul tastes, more preferably irritation, burning and bitterness.

6. Dosage form according to any one of claims 1 to 5, **characterized in that** the one or more organoleptically disturbing sensations is/are induced by
i) the nicotine; and/or
ii) one or more excipients of the dosage form, optionally the one or more excipients encompass/es one or more buffers selected from the group consisting of carbonate (including bicarbonate or sesquicarbonate), glycinate, different phosphate systems such as trisodium phosphate, disodium hydrogen phosphate; and tripotassium phosphate, dipotassium hydrogen phosphate, glycerophosphate or citrate of an alkali metal (such as potassium or sodium, or ammonium), e.g. trisodium and tripotassium citrate, different hydroxides, amino acids, and mixtures thereof, and other excipients that may induce organoleptically disturbing sensations.

7. Dosage form according to any one of claims 1 to 6, **characterized in that** the nicotine is in a form selected from a group consisting of a nicotine salt, the free base form of nicotine, a nicotine derivative, such as a nicotine cation exchanger, a nicotine inclusion complex, nicotine in any non-covalent binding, nicotine bound to zeolites, nicotine bound to cellulose including micro-crystalline cellulose, or starch micro-spheres and mixtures thereof.

8. Dosage form according to any one of claims 1 to 7, **characterized in that** the at least one component for reduction of one or more organoleptically disturbing sensations comprises or consists of one or more
i) flavoring agents, optionally wherein the one or more flavoring agents are selected from the group consisting of natural or synthetic flavouring or aromatizing agents and may be added as liquids and/or as powder, and that they may be selected from essential oils including distillations, solvent extractions, or cold expressions of chopped flowers, leaves, peel or pulped whole fruit comprising mixtures of alcohols, esters, aldehydes and lactones; essences including either diluted solutions of essential oils, or mixtures of synthetic chemicals blended to match the natural flavor of the fruit, (e.g. strawberry, raspberry, black currant, banana, melon, cherry, passion fruit, pineapplee, peach, blackberry, mango, papaya, guava, cranberry, cloudberry, violet, pomegranate, pear, apple); artificial and natural flavors of brews and liquors, (e.g. cognac, whisky, rum, gin, sherry, port, and wine); tobacco, coffee, tea, cocoa, and mint; fruit juices including expelled juice from washed, scrubbed fruits such as lemon, orange, and lime and other citric fruits; spear mint, pepper mint, lemon balm, wintergreen; cinnamon, cacoe/cocoa, vanilla, liquorice, menthol, eucalyptus, aniseeds, nuts (e.g. peanuts, coconuts, hazelnuts, chestnuts, walnuts, colanuts), almonds, raisins and ginger; and powder and flour, and combinations thereof; and/or
ii) sweeteners, optionally wherein the one or more sweeteners are selected from the group consisting of synthetic or natural sugars, i.e. any form of carbohydrates suitable for use as sweetener, as well as so called artificial sweeteners such as saccharin, sodium saccharin, aspartame, acesulfame or Acesulfame K, potassium acesulfame, thaumatin, glycyrrhizin, sucralose, dihydrochalcone, miraculin, monellin, stevside, neotame, N-substituted APM derivatives, cyclamic acid and its salts and alitame, the group consisting of sugar alcohols, such as sorbitol, xylitol, single sugars including sugars extracted from sugar cane and sugar beet (sucrose), dextrose (also called glucose), fructose (also called leavulose), and lactose (also called milk sugar); sorbitol, mannitol, glycerol, xylitol, erythritol, maltitol syrup (or hydrogenated starch hydrolyzate), isomalt, lactitol; and mixtures of sugars including glucose syrup, (e.g. starch hydrolysates, containing a mixture of dextrose, maltose and a range of complex sugars), invert sugar syrup, (e.g. sucrose inverted by invertase (also called sucrase or sacchrase) containing a mixture of dextrose and fructose), high sugar content syrups such as treacle and honey containing a mixture of particular leavulose, dextrose, maltose, lactitole, sucrose, resins, dextrin and higher sugars; and malt or malt extracts, and combinations thereof; and/or
iii) flavoring agents and one or more sweeteners.

9. Dosage form according to any one of claims 1 to 8, **characterized in that** the one or more film-forming polymers is/are selected from the group consisting of cellulose ethers, such as hydroxy propyl methyl cellulose (HPMC), methyl hydroxy ethyl cellulose (MHEC), hydroxy propyl cellulose (HPC), hydroxyethyl cellulose (HEC), ethyl hydroxyl ethyl cellulose (EHEC), and other film forming polymers, such as methacrylic acid copolymer-type C, sodium carboxy methyl cellulose, polydextrose, polyethylene glycols, acrylate polymers, such as polyvinyl acrylate (PVA)), polyvinyl alcohol-polyethylene glycol graft copolymers, complex of polyvinylpyrrolidone (PVP), such as povidone, polyvinyl alcohol, microcrystalline cellulose, carrageenan, pregelatinized starch, polyethylene glycol, and combinations thereof.

10. Dosage form according to any preceding claim, **characterized in that** it further comprises
one or more plasticizers, such as glycerol, propylene glycol, polyethylene glycol (PEG 200-6000), organic esters such as triacetin (glyceryl triacetate), triethyl citrate, diethyl phtalate, dibutyl phtalate, dibutyl sebacete, acetyltriethyl citrate, acethyltributyl citrate, tributyl citrate and oils/glycerides, such as fractionated coconut oil, castor oil and distilled acetylated monoglycerides, and/or
one or more surfactants, such as polysorbates derived from PEG-ylated sorbitan esterified with fatty acids such as Polysorbate 20 (Polyoxyethylene (20) sorbitan monolaurate), Polysorbate 40 (Polyoxyethylene (20) sorbitan monopalmitate), Polysorbate 60 (Polyoxyethylene (20) sorbitan monostearate), Polysorbate 80 (Polyoxyethylene (20) sorbitan monooleate) (e.g. Tween 80, Tween 40, Tween 20), sodium lauryl sulphate (SLS), poloxamer surfactants i.e. surfactants based on ethylene oxide - propylene oxide block copolymers and other surfactants with high HLB-value;
and/or
one or more anti-tacking agents or glidants, such as talc, magnesium stearate, kaolin, colloidal silicon dioxide and glyceryl monostearate, and/or
one or more coloring agents, and/or
one or more opacifiers, and/or
one or more glossing agents, and/or
one or more pore forming agents, and/or
one or more excipient stabilizers.

11. Dosage form according to any one of claims 1 to 10, **characterized in that**
i) the at least one film coating dissolves or disintegrates rapidly upon administration to the oral cavity, preferably in less than 2 minutes, more preferably in less than 1 minute and most preferably in less than 30 seconds, from the moment of administration; and/or
ii) said dosage form releases the nicotine within 30 minutes upon administration to the oral cavity, preferably within 20 minutes, from the moment of administration; and/or
iii) the reduction of one or more organoleptically disturbing sensations does not noticeably deteriorate the pharmaceutical effect of the nicotine and/or the API.

12. Dosage form according to any one of claims 1 to 11, **characterized in that** a unit dose thereof has a core comprising
- nicotine in an amount corresponding to 0.5 mg to 12 mg nicotine base,
- sorbitol 0 - 99.15 % (w/w),
- xylitol 0 - 99.15 % (w/w),
- mannitol 0 - 99.15 % (w/w),
- sodium bicarbonate 0 - 1.0 % (w/w),
- sodium carbonate 0 - 1.0 % (w/w),
- flavor 0.05 - 2.5 % (w/w),
- high intensity sweetener or combination of high intensity sweeteners 0 - 0.25 % (w/w),
- magnesium stearate 0.5 - 2.5 % (w/w).

13. Dosage form according to any one of claims 1 to 12, **characterized in that** the coating comprises
i) film-forming polymer around 80 % (w/w), surfactant around 0.3% (w/w), high intensity sweetener or combination of high intensity sweeteners around 8 % (w/w), flavor around 12 % (w/w); or
ii) film-forming polymer 80.5 % (w/w),
plasticizer 8 % (w/w)
surfactant 0.1 % (w/w),
coloring component 6 % (w/w)
high intensity sweetener or combination of high intensity sweeteners 4 % (w/w),
flavor 1,25 % (w/w); or
iii) film-forming polymer from 44.5 to 97.0 % (w/w),
plasticizer from 0 to 25.0 % (w/w),
surfactant from 0 to 0.5 % (w/w),
coloring component from 0 to 10 % (w/w),
high intensity sweetener or combination of high intensity sweeteners from 0.5 to 10 % (w/w),
flavor from 2,5 to 10 % (w/w).

14. Dosage form according to any one of claims 1 to 11, **characterized in that** a unit dose comprises
i) the below amounts in mg in the core and the coating respectively, whereby the respective amounts may vary within an interval of ± 15 % (w/w), preferably within an interval of ± 5 % (w/w)
**Core**
| | |
|---|---|
| Mannitol | 175.78 |
| Potassium hydrogencarbonate | 0.45 |
| Sodium carbonate | 3.67 |
| Sodium alginate | 10.30 |
| Xanthan gum | 1.99 |
| Calcium polycarbophil | 5.13 |
| Nicotine resin complex | 22.22 |
| Potassium hydrogencarbonate | 0.58 |
| Sodium carbonate anhydrous | 4.63 |
| Aspartame | |
| Acesulfame Potassium | 1.50 |
| Mint flavor | 21.25 |
| Magnesium stearate | 2.50 |
| Total weight of tablet core mg | 250 |
**Coating**
| | |
|---|---|
| Hydroxypropyl methylcellulose | 4.83 |
| Titan dioxide | 0.19 |
| Polyethyleneq,jygol 400* | 0.094 |
| Sodium lauryl sulfate | 0.019 |
| Aspartame | 0.31 |
| Acesulfame Potassium | 0.19 |
| Mint flavour | 3.00 |
; or
ii) the below amounts in mg in the core and the coating respectively, whereby the respective amounts may vary within an interval of ± 15 % (w/w), preferably within an interval of ± 5 % (w/w)
**Core**
| | |
|---|---|
| Mannitol | 1034.85 |
| Potassium hydrogencarbonate | 2.80 |
| Sodium carbonate | 22.75 |
| Sodium alginate | 63.70 |
| Xanthan gum | 12.25 |
| Calcium polycarbophil | 31.73 |
| Nicotine resin complex | 22.22 |
| Aspartame | 6.00 |
| Mint flavor | 1.2 |
| Magnesium stearate | 2.50 |
| Total weight of tablet core mg | 1200 |
**Coating**
| | |
|---|---|
| Hydroxypropyl methylcellulose | 23.19 |
| Titan dioxide | 0.90 |
| Polyethyleneqlypol 400 | 0.45 |
| Sodium lauryl sulfate | 0.090 |
| Aspartame | 1.47 |
| Acesulfame Potassium | 0.90 |
| Mint flavor | 3.00 |

15. Dosage form according to any of claims 1 - 14 for use in:
i) therapy wherein the therapy is treatment of a disease selected from the group consisting of tobacco or nicotine dependence, Alzheimer's disease, Crohn's disease, Parkinson's disease, Tourette's syndrome, ulcerous colitis and post-smoking-cessation weight gain, specifically tobacco or nicotine dependence; or
ii) a method of treating tobacco or nicotine dependence, Alzheimer's disease, Crohn's disease, Parkinson's disease, Tourette's syndrome, ulcerous colitis and post-smoking-cessation weight gain, the method comprising administering the dosage form according to any of claims 1 - 14 orally to the patient, optionally wherein the dosage form is retained in the oral cavity following administration to the patient such that the nicotine is absorbed by the mucosa of the oral cavity and/or by the mucosa of the pharynx.

16. Therapy system comprising a dosage form according to any of claims 1 - 15 comprising nicotine in any form.

## Patentansprüche

1. Feste pharmazeutische Dosierungsform zur Freisetzung von Nicotin in der Mundhöhle, umfassend einen von mindestens einem Filmüberzug umkapselten Kern, wobei der Kern Nicotin umfasst und wobei der Filmüberzug mindestens ein filmbildendes Polymer und mindestens eine Komponente zur Verminderung von einer oder mehreren organoleptisch störenden Empfindungen umfasst und wobei der mindestens eine Filmüberzug kein Nicotin und keinen Puffer umfasst, wobei es sich bei der Dosierungsform um eine Lutschtablette, eine sublinguale Tablette, eine bukkale Tablette oder eine im Mund zerfallende Tablette handelt.

2. Dosierungsform nach Anspruch 1, **dadurch gekennzeichnet, dass** sie mindestens einen weiteren pharmazeutischen Wirkstoff wie ein Zinksalz oder einen Zinkkomplex umfasst.

3. Dosierungsform nach Anspruch 1 oder 2, **dadurch gekennzeichnet, dass** das Nicotin nach der Verabreichung im Wesentlichen von der Schleimhaut der Mundhöhle und/oder der Schleimhaut des Rachens resorbierbar ist.

4. Dosierungsform nach einem der Ansprüche 1 bis 3, **dadurch gekennzeichnet, dass** der mindestens eine Filmüberzug vorzugsweise eine Dicke von 10 bis 500 Mikron, besonders bevorzugt von 20 bis 250 Mikron und am meisten bevorzugt von 30 bis 150 Mikron aufweist und/oder
der Kern vorzugsweise ein Gewicht von 50 mg bis 2000 mg, besonders bevorzugt von 90 mg bis 1200 mg aufweist und/oder
der Filmüberzug vorzugsweise ein Gewicht von 1% bis 15% des Gewichts des Kerns aufweist.

5. Dosierungsform nach einem der Ansprüche 1 bis 4, **dadurch gekennzeichnet, dass** die organoleptisch störende(n) Empfindung(en) aus der aus Reizung, Schärfe, Geschmacksveränderung und Geschmacksblockade, brennenden, adstringierenden, bitteren und kribbelnden Gefühlen und Beigeschmäcken wie sauren, salzigen, metallischen, seifigen, modrigen, schwefligen, penetranten, fettigen und faulen Geschmäcken, vorzugsweise Reizung, Schärfe, brennenden, bitteren und kribbelnden Gefühlen, Beigeschmäcken wie sauren, salzigen, metallischen, seifigen, fettigen und faulen Geschmäcken, besonders bevorzugt Reizung, Brennen und bitterem Geschmack bestehenden Gruppe ausgewählt ist/sind.

6. Dosierungsform nach einem der Ansprüche 1 bis 5, **dadurch gekennzeichnet, dass** die eine oder die mehreren organoleptisch störenden Empfindungen induziert ist/sind durch
i) das Nicotin und/oder
ii) einen oder mehrere Exzipienten der Dosierungsform, wobei der eine oder die mehreren Exzipienten gegebenenfalls einen oder mehrere Puffer ausgewählt aus der Gruppe bestehend aus Carbonat (einschließlich Hydrogencarbonat oder Sesquicarbonat), Glycinat, verschiedenen Phosphatsystemen wie Trinatriumphosphat, Dinatriumhydrogenphosphat und Trikaliumphosphat, Dikaliumhydrogenphosphat, Glycerophosphat oder Citrat eines Alkalimetalls (wie Kalium oder Natrium, oder Ammonium), z.B. Trinatrium- und Trikaliumcitrat, verschiedenen Hydroxiden, Aminosäuren und Mischungen davon und anderen Exzipienten, die organoleptisch störende Empfindungen induzieren können, umfasst/umfassen.

7. Dosierungsform nach einem der Ansprüche 1 bis 6, **dadurch gekennzeichnet, dass** das Nicotin in einer Form ausgewählt aus einer Gruppe bestehend aus einem Nicotinsalz, der freien Basenform von Nicotin, einem Nicotinderivat wie einem Nicotinkationenaustauscher, einem Nicotineinschlusskomplex, Nicotin in einer nichtkovalenten Bindung, an Zeolithe gebundenem Nicotin, an Cellulose einschließlich mikrokristalliner Cellulose oder Stärkemikrokügelchen gebundenem Nicotin und Mischungen davon vorliegt.

8. Dosierungsform nach einem der Ansprüche 1 bis 7, **dadurch gekennzeichnet, dass** die mindestens eine Komponente zur Reduktion einer oder mehrerer organoleptisch störender Empfindungen ein oder mehrere der folgenden umfasst oder daraus besteht:
i) Geschmacksstoffe, wobei der eine oder die mehreren Geschmacksstoffe gegebenenfalls ausgewählt sind aus der Gruppe bestehend aus natürlichen oder synthetischen Geschmäcken oder Aromastoffen und als Flüssigkeiten und/oder als Pulver zugesetzt werden können und ausgewählt sein können aus essenziellen Ölen einschließlich Destillaten, Lösungsmittelextrakten oder kalten Exprimaten gehackter Blütenblätter, Blätter, Schalen oder zu Brei verarbeiteter ganzer Früchte mit Mischungen von Alkoholen, Estern, Aldehyden und Lactonen, Essenzen, die entweder verdünnte Lösungen essenzieller Öle oder Mischungen synthetischer Chemikalien, die so gemischt werden, dass die dem natürlichen Geschmack der Frucht entsprechen, einschließen (z.B. Erdbeere, Himbeere, Johannisbeere, Banane, Melone, Kirsche, Passionsfrucht, Ananas, Pfirsich, Brombeere, Mango, Papaya, Guave, Cranberry, Moltebeere, Veilchen, Granatapfel, Birne, Apfel), künstliche und natürliche Geschmäcke von Gebräuen und Likören (z.B. Kognak, Whisky, Rum, Gin, Sherry, Port und Wein), Tabak, Kaffee, Tee, Kakao und Minze, Fruchtsäfte einschließlich ausgepresstem Saft aus gewaschenen, gesäuberten Früchten wie Zitrone, Orange und Limone und anderen Zitrusfrüchten, grüne Minze, Pfefferminze, Zitronenmelisse, Wintergrün, Zimt, Kakao, Vanille, Lakritze, Menthol, Eukalyptus, Anis, Nüssen (z.B. Erdnüssen, Kokosnüssen, Haselnüssen, Kastanien, Walnüssen, Kolanüssen), Mandeln, Rosinen und Ingwer, sowie Pulver und Mehl und Kombinationen davon, und/oder
ii) Süßstoffe, wobei der eine oder die mehreren Süßstoffe gegebenenfalls ausgewählt sind aus der Gruppe bestehend aus synthetischen oder natürlichen Zuckern, d.h. jeglicher Form von für die Verwendung als Süßstoff geeigneten Kohlenhydraten, sowie sogenannten künstlichen Süßstoffen wie Saccharin, Natriumsaccharin, Aspartam, Acesulfam oder Acesulfam K, Kaliumacesulfam, Thaumtin, Glycyrrhizin, Sucralose, Dihydrochalcon, Miraculin, Monellin, Stevsid, Neotam, N-substituierten APM-Derivaten, Cyclamsäure und deren Salzen und Alitam, der aus Zuckeralkoholen wie Sorbit und Xylit bestehenden Gruppe, einzelnen Zuckern einschließlich aus Zuckerrohr und Zuckerrübe extrahiertem Zucker (Saccharose), Dextrose (auch als Glucose bezeichnet), Fructose (auch als Leavulose bezeichnet) und Lactose (auch als Milchzucker bezeichnet), Sorbit, Mannit, Glycerin, Xylit, Erythrit, Maltitsirup (oder hydriertes Stärkehydrolisat), Isomalt, Lactit und Zuckermischungen einschließlich Glucosesirup (z.B. Stärkehydrolysate, die eine Mischung von Dextrose, Maltose und einer Reihe von komplexen Zuckern enthalten), Invertzuckersirup (z.B. durch Invertase invertierte Saccharose (auch als Sucrase oder Sacchrase bezeichnet), die eine Mischung von Dextrose und Fructose enthält), Sirupe mit hohem Zuckergehalt wie Melasse und Honig, die eine Mischung insbesondere von Leavulose, Dextrose, Maltose, Lactit, Saccharose, Harzen, Dextrin und höheren Zuckern enthalten, und Malz oder Malzextrakte und Kombinationen davon, und/oder
iii) Geschmacksstoffe und ein oder mehrere Süßstoffe.

9. Dosierungsform nach einem der Ansprüche 1 bis 8, **dadurch gekennzeichnet, dass** das eine oder die mehreren filmbildenden Polymere ausgewählt ist/sind aus der Gruppe bestehend aus Celluloseethern wie Hydroxypropylmethylcellulose (HPMC), Methylhydroxyethylcellulose (MHEC), Hydroxypropylcellulose (HPC), Hydroxyethylcellulose (HEC), Ethylhydroxyethylcellulose (EHEC) und anderen filmbildenden Polymeren wie Methacrylsäurecopolymer vom Typ C, Natriumcarboxymethylcellulose, Polydextrose, Polyethylenglykole, Acrylatpolymere wie Polyvinylacrylat (PVA), Polyvinylalkohol-Polyethylenglykol-Pfropfcopolymere, ein Komplex von Polyvinylpyrrolidon (PVP) wie Povidon, Polyvinylalkohol, mikrokristalliner Cellulose, Carrageen, vorgelatinisierter Stärke, Polyethylenglykol und Kombinationen davon.

10. Dosierungsform nach einem der vorhergehenden Ansprüche, **dadurch gekennzeichnet, dass** sie weiterhin Folgendes umfasst:
einen oder mehrere Weichmacher wie Glycerin, Propylenglykol, Polyethylenglykol (PEG 200-6000), organische Ester wie Triacetin (Glyceryltriacetat), Triethylcitrat, Diethylphtalat, Dibutylphtalat, Dibutylsebacat, Acetyltriethylcitrat, Acethyltributylcitrat, Tributylcitrat und Öle/Glyceride wie fraktioniertes Kokosnussöl, Rizinusöl und destillierte acetyllierte Monoglyceride, und/oder
einen oder mehrere Tenside wie Polysorbat abgeleitet von PEG-yliertem Sorbitan verestert mit Fettsäuren, wie Polysorbat 20 (Polyoxyethylen(20)-sorbitanmonolaurat), Polysorbat 40 (Poloxy-ethylen(20)-sorbitanmonopalmitat), Polysorbat 60 (Polyoxyethylen(20)-sorbitanmonostearat), Polysorbat 80 (Polyoxyethylen(20)-sorbitanmonooleat) (z.B. Tween 80, Tween 40, Tween 20), Natriumlaurylsulfat (SLS), Poloxamer-Tensiden, d. h. Tensiden basierend auf Ethylenoxid-Propylenoxid-Blockcopolymeren, und andere Tenside mit einem hohem HLB-Wert,
und/oder
ein oder mehrere Antihaftmittel oder Gleitmittel wie Talk, Magnesiumstearat, Kaolin, kolloidales Siliciumdioxid und Glycerylmonostearat und/oder
ein oder mehrere Farbstoffe und/oder
ein oder mehrere Trübungsmittel und/oder
ein oder mehrere Glanzmittel und/oder
ein oder mehrere porenbildende Mittel und/oder
ein oder mehrere Stabilisatorexzipienten.

11. Dosierungsform nach einem der Ansprüche 1 bis 10, **dadurch gekennzeichnet, dass**
i) der mindestens eine Filmüberzug bei der Verabreichung an die Mundhöhle sich schnell auflöst oder zerfällt, vorzugsweise in weniger als 2 Minuten, besonders bevorzugt in weniger als 1 Minute und am meisten bevorzugt in weniger als 30 Sekunden, vom Zeitpunkt der Verabreichung, und/oder
ii) die Dosierungsform das Nicotin innerhalb von 30 Minuten nach Verabreichung an die Mundhöhle freisetzt, vorzugsweise innerhalb von 20 Minuten, vom Zeitpunkt der Verabreichung, und/oder
iii) die Verminderung der einen oder mehreren organoleptisch störenden Empfindungen die pharmazeutische Wirkung des Nicotins und/oder des API nicht merkenswert herabsetzt.

12. Dosierungsform nach einem der Ansprüche 1 bis 11, **dadurch gekennzeichnet, dass** eine Einheitsdosis davon einen Kern aufweist, umfassend
- Nicotin in einer Menge, die 0,5 mg bis 12 mg Nicotinbase entspricht,
- Sorbit 0 - 99,15 Gew.-%,
- Xylit 0 - 99,15 Gew.-%,
- Mannit 0 - 99,15 Gew.-%,
- Natriumhydrogencarbonat 0 - 1,0 Gew.-%,
- Natriumcarbonat 0 - 1,0 Gew.-%,
- Geschmack 0,05 - 2,5 Gew.-%,
- hochintensiver Süßstoff oder Kombination hochintensiver Süßstoffe 0 - 0,25 Gew.-%,
- Magnesiumstearat 0,5 - 2,5 Gew.-%.

13. Dosierungsform nach einem der Ansprüche 1 bis 12, **dadurch gekennzeichnet, dass** der Überzug Folgendes umfasst:
i) filmbildendes Polymer etwa 80 Gew.-%, Tensid etwa 0,3 Gew.-%,
hochintensiver Süßstoff oder Kombination hochintensiver Süßstoffe etwa 8 Gew.-%, Geschmack etwa 12 Gew.-% oder
ii) filmbildendes Polymer 80,5 Gew._% Weichmacher 8 Gew.-%,
Tenside 0,1 Gew.-%,
farbgebende Komponente 6 Gew.-%, hochintensiver Süßstoff oder Kombination hochintensiver Süßstoffe 4 Gew.-%,
Geschmack 1,25 Gew.-% oder
iii) filmbildendes Polymer 44,5 bis 97,0 Gew.-%, Weichmacher 0 bis 25,0 Gew.-%,
Tensid 0 bis 0,5 Gew.-%,
farbgebende Komponente 0 bis 10 Gew.-%, hochintensiver Süßstoff oder Kombination hochintensiver Süßstoffe 0,5 bis 10 Gew.-%, Geschmacksstoff 2,5 bis 10 Gew.-%.

14. Dosierungsform nach einem der Ansprüche 1 bis 11, **dadurch gekennzeichnet, dass** eine Einheitsdosis Folgendes umfasst:
i) die unten angeführten Mengen in mg im Kern bzw. Überzug, wobei die jeweiligen Mengen in einem Intervall von ± 15 Gew.-%, vorzugsweise in einem Intervall von ± 5 Gew.-%, variieren können:
**Kern**
| | |
|---|---|
| Mannit | 175,78 |
| Kaliumhydrogencarbonat | 0,45 |
| Natriumcarbonat | 3,67 |
| Natriumalginat | 10,30 |
| Xanthan | 1,99 |
| Calciumpolycarbophil | 5,13 |
| Nicotin-Harz-Komplex | 22,22 |
| Kaliumhydrogencarbonat | 0,58 |
| Natriumcarbonat wasserfrei | 4,63 |
| Aspartam | |
| Acesulfam-Kalium | 1,50 |
| Minzgeschmack | 21,25 |
| Magnesiumstearat | 2,50 |
| Gesamtgewicht des Tablettenkerns mg | 250 |
**Überzug**
| | |
|---|---|
| Hydroxypropylmethylcellulose | 4,83 |
| Titandioxid | 0,19 |
| Polyethylenglykol 400* | 0,094 |
| Natriumlaurylsulfat | 0,019 |
| Aspartam | 0,31 |
| Acesulfam-Kalium | 0,19 |
| Minzgeschmack | 3,00 |
oder
ii) die unten angeführten Mengen in mg im Kern bzw. Überzug, wobei die jeweiligen Mengen in einem Intervall von ± 15 Gew.-%, vorzugsweise in einem Intervall von ± 5 Gew.-%, variieren können:
**Kern**
| | |
|---|---|
| Mannit | 1034,85 |
| Kaliumhydrogencarbonat | 2,80 |
| Natriumcarbonat | 22,75 |
| Natriumalginat | 63,70 |
| Xanthan | 12,25 |
| Calciumpolycarbophil | 31,73 |
| Nicotin-Harz-Komplex | 22,22 |
| Aspartam | 6,00 |
| Minzgeschmack | 1,2 |
| Magnesiumstearat | 2,50 |
| Gesamtgewicht des Tablettenkerns mg | 1200 |
**Überzug**
| | |
|---|---|
| Hydroxypropylmethylcellulose | 23,19 |
| Titandioxid | 0,90 |
| Polyethylenglykol 400 | 0,45 |
| Natriumlaurylsulfat | 0,090 |
| Aspartam | 1,47 |
| Acesulfam-Kalium | 0,90 |
| Minzgeschmack | 3,00 |

15. Dosierungsform nach einem der Ansprüche 1-14 zur Verwendung in:
i) der Therapie, wobei es sich bei der Therapie um die Behandlung einer Krankheit ausgewählt aus der Gruppe bestehend aus Tabak- bzw. Nicotinabhängigkeit, Alzheimer-Krankheit, Morbus-Crohn, Parkinson-Krankheit, Tourette-Syndrom, Colitis ulcerosa und Gewichtszunahme nach Aufhören mit dem Rauchen, speziell Tabak- bzw. Nicotinabhängigkeit, handelt oder
ii) einem Verfahren zur Behandlung von Nicotinabhängigkeit, Alhzeimer-Krankheit, Morbus Crohn, Parkinson-Krankheit, Tourette-Syndrom, Colitis ulcerosa und Gewichtszunahme nach Aufhören mit dem Rauchen, wobei das Verfahren die orale Verabreichung der Dosierungsform nach einem der Ansprüche 1-14 an den Patienten umfasst, wobei die Dosierungsform nach der Verabreichung an den Patienten gegebenenfalls in der Mundhöhle zurückgehalten wird, so dass das Nicotin über die Schleimhaut der Mundhöhle und/oder über die Schleimhaut des Rachens resorbiert wird.

16. Therapiesystem, umfassend eine Dosierungsform nach einem der Ansprüche 1 - 15, die Nicotin in einer beliebigen Form umfasst.

## Revendications

1. Forme de dosage pharmaceutique solide pour la libération de nicotine dans la cavité buccale comprenant un noyau encapsulé par au moins un revêtement en film, le noyau comprenant de la nicotine et le revêtement en film comprenant au moins un polymère filmogène et au moins un composant pour la réduction d'une ou plusieurs sensations de perturbation organoleptique, et l'au moins un revêtement en film étant exempt de nicotine et exempt de tampon ;
ladite forme de dosage étant une pastille, un comprimé sublingual, un comprimé buccal, ou un comprimé pour désintégration orale.

2. Forme de dosage selon la revendication 1, **caractérisée en ce qu'**elle comprend au moins un ingrédient pharmaceutique actif supplémentaire, tel qu'un sel de zinc ou un complexe de zinc.

3. Forme de dosage selon la revendication 1 ou 2, **caractérisée en ce que** lors de l'administration, ladite nicotine est essentiellement absorbable par la muqueuse de la cavité buccale et/ou par la muqueuse du pharynx.

4. Forme de dosage selon l'une quelconque des revendications 1 à 3, **caractérisée en ce que** l'au moins un revêtement en film possède préférablement une épaisseur de 10 à 500 microns, plus préférablement de 20 à 250 microns, et le plus préférablement de 30 à 150 microns et/ou,
le noyau possède préférablement un poids de 50 mg à 2 000 mg, plus préférablement de 90 mg à 1 200 mg et/ou
le revêtement en film possède préférablement un poids allant de 1 % à 15 % du poids du noyau.

5. Forme de dosage selon l'une quelconque des revendications 1 à 4, **caractérisée en ce que** la ou les sensation(s) de perturbation organoleptique est/sont choisie(s) dans le groupe constitué par une irritation, une âcreté, une altération du goût et un blocage du goût, des sensations de brûlure, d'astringence, d'amertume et de picotement, et des arrière-goûts tels que des goûts acides, salés, métalliques, savonneux, moisis, sulfureux, piquants, gras et nauséabonds, plus préférablement une irritation, une brûlure et une amertume.

6. Forme de dosage selon l'une quelconque des revendications 1 à 5, **caractérisée en ce que** la ou les sensation(s) de perturbation organoleptique est/sont induite(s) par
i) la nicotine ; et/ou
ii) un ou plusieurs excipients de la forme de dosage, éventuellement le ou les excipients englobe(nt) un ou plusieurs tampons choisis dans le groupe constitué par un carbonate (y compris bicarbonate ou sesquicarbonate), un glycinate, différents systèmes de phosphate tels qu'un phosphate trisodique, un hydrogénophosphate disodique ; et un phosphate tripotassique, un hydrogénophosphate dipotassique, un glycérophosphate ou un citrate d'un métal alcalin (tel que potassium ou sodium, ou ammonium), par ex. un citrate trisodique et tripotassique, différents hydroxydes, des acides aminés, et des mélanges correspondants, et d'autres excipients qui peuvent induire des sensations de perturbation organoleptique.

7. Forme de dosage selon l'une quelconque des revendications 1 à 6, **caractérisée en ce que** la nicotine se trouve sous une forme choisie dans un groupe constitué par un sel de nicotine, la forme de base libre de la nicotine, un dérivé de nicotine, tel qu'un échangeur de cation de nicotine, un complexe d'inclusion de nicotine, la nicotine dans une quelconque liaison non covalente, la nicotine liée à des zéolithes, la nicotine liée à une cellulose y compris une cellulose microcristalline, et des microsphères d'amidon et des mélanges correspondants.

8. Forme de dosage selon l'une quelconque des revendications 1 à 7, **caractérisée en ce que** l'au moins un composant pour la réduction d'une ou plusieurs sensations de perturbation organoleptique comprend un ou plusieurs, ou est constitué d'un ou plusieurs
i) agents d'arôme, éventuellement l'agent ou les agents d'arôme étant choisis dans le groupe constitué par des agents d'arôme ou aromatisants naturels ou synthétiques et pouvant être ajoutés comme liquide et/ou comme poudre, et pouvant être choisis parmi des huiles essentielles y compris des distillations, des extractions au solvant, ou des expressions à froid de fleurs hachées, de feuilles, de pelure, ou de fruits entiers en pâte, , comprenant des mélanges d'alcools, d'esters, d'aldéhydes et de lactones ; des essences y compris soit des solutions diluées d'huiles essentielles, soit des mélanges de produits chimiques synthétiques assemblés pour correspondre à l'arôme naturel du fruit, (par ex. fraise, framboise, cassis, banane, melon, cerise, fruit de la passion, ananas, pêche, mûre, mangue, papaye, goyave, airelle, plaquebière, violette, grenade, poire, pomme) ; des arômes artificiels et naturels de brassins et de liqueurs, (par ex. cognac, whiskey, rhum, gin, sherry , porto, et vin) ; tabac, café, thé, cacao, et menthe ; des jus de fruits y compris le jus expulsé de fruits lavés et frottés tels que citron, orange, et citron vert et autres agrumes ; menthe verte, menthe poivrée, mélisse, gaulthérie ; cannelle, cacaos/cacao, vanille, réglisse, menthol, eucalyptus, graines d'anis, noix (par ex. arachides, noix de coco, noisettes, châtaignes, noix, noix de cola), amandes, raisins secs et gingembre ; et poudre et farine, et des combinaisons correspondantes ; et/ou
ii) des édulcorants, éventuellement l'édulcorant ou les édulcorants étant choisis dans le groupe constitué par des sucres synthétiques ou naturels, c'est-à-dire une quelconque forme de glucide appropriée pour une utilisation en tant qu'édulcorant, ainsi que des dénommés édulcorants artificiels tels que saccharine, saccharine sodique, aspartame, acésulfame ou Acésulfame K, acésulfame potassique, thaumatine, glycyrrhizine, sucralose, dihydrochalcone, miraculine, monelline, stévioside, néotame, dérivés d'APM N-substitués, acide cyclamique et ses sels et alitame, le groupe constitué par des alcools de sucre, tels que sorbitol, xylitol, sucres simples y compris des sucres extraits de canne à sucre et de betterave à sucre (saccharose), dextrose (également appelé glucose), fructose (également appelé lévulose), et lactose (également appelé sucre du lait) ; sorbitol, mannitol, glycérol, xylitol, érythritol, sirop de maltitol (ou hydrolysat d'amidon hydrogéné), isomalt, lactitol ; et des mélanges de sucre y compris le sirop de glucose, (par ex. des hydrolysats d'amidon, contenant un mélange de dextrose, maltose et une variété de sucres complexes), le sirop de sucre inverti, (par ex. saccharose inverti par une invertase (également appelée sucrase or saccharase) contenant un mélange de dextrose et fructose), des sirops à teneur élevée en sucre tels que mélasse et miel contenant un mélange de sucres de type lévulose, dextrose, maltose, lactitol, saccharose, résines, dextrine et de sucres supérieurs particuliers ; et malt ou extraits de malt, et des combinaisons correspondantes ; et/ou
iii) des agents d'arôme et un ou plusieurs édulcorants.

9. Forme de dosage selon l'une quelconque des revendications 1 à 8, **caractérisée en ce que** le ou les polymères filmogènes est/sont choisi(s) dans le groupe constitué par des éthers de cellulose, tels qu'une hydroxypropylméthylcellulose (HPMC), une méthylhydroxyéthylcellulose (MHEC), une hydroxypropylcellulose (HPC), une hydroxyéthylcellulose (HEC), une éthylhydroxyéthylcellulose (EHEC), et d'autres polymères filmogènes, tels qu'un copolymère d'acide méthacrylique de type C, une carboxyméthylcellulose sodique, un polydextrose, des polyéthylèneglycols, des polymères d'acrylate, tels qu'un poly(acrylate de vinyle) (PVA), des copolymères greffés de poly(alcool vinylique)-polyéthylèneglycol, un complexe de polyvinylpyrrolidone (PVP), tel qu'une povidone, un poly(alcool vinylique), une cellulose microcristalline, un carraghénane, un amidon pré-gélifié, un polyéthylèneglycol, et des combinaisons correspondantes.

10. Forme de dosage selon une quelconque revendication précédente, **caractérisée en ce qu'**elle comprend en outre
un ou plusieurs plastifiants, tels que le glycérol, le propylèneglycol, un polyéthylèneglycol (PEG 200-6000), des esters organiques tels que la triacétine (triacétate de glycéryle), le citrate de triéthyle, le phtalate de diéthyle, le phtalate de dibutyle, le sébaçate de dibutyle, le citrate d'acétyltriéthyle, le citrate d'acétyltributyle, le citrate de tributyle et des huiles/glycérides, tels que l'huile de noix de coco fractionnée, l'huile de ricin et des monoglycérides acétylés distillés, et/ou
un ou plusieurs tensioactifs, tels que des polysorbates issus de sorbitane PEG-ylé estérifié avec des acides gras tels que Polysorbate 20 (monolaurate de polyoxyéthylène (20) sorbitane), Polysorbate 40 (monopalmitate de polyoxyéthylène (20) sorbitane), Polysorbate 60 (monostéarate de polyoxyéthylène (20) sorbitane), Polysorbate 80 (monooléate de polyoxyéthylène (20) sorbitane) (par ex. Tween 80, Tween 40, Tween 20), le laurylsulfate de sodium (SLS), des tensioactifs de type poloxamère c'est-à-dire des tensioactifs à base de copolymères à blocs d'oxyde d'éthylène-oxyde de propylène et d'autres tensioactifs dotés d'une valeur HLB élevée ;
et/ou
un ou plusieurs agents antiadhérents ou glissants, tels que le talc, le stéarate de magnésium, le kaolin, le dioxyde de silicium colloïdal et le monostéarate de glycéryle, et/ou
un ou plusieurs agents colorants, et/ou
un ou plusieurs opacifiants, et/ou
un ou plusieurs agents de brillance, et/ou
un ou plusieurs agents porogènes, et/ou
un ou plusieurs stabilisants d'excipient.

11. Forme de dosage selon l'une quelconque des revendications 1 à 10, **caractérisée en ce que**
i) l'au moins un revêtement en film se dissout ou se désintègre rapidement lors d'une administration à la cavité buccale, préférablement en moins de 2 minutes, plus préférablement en moins de 1 minute et le plus préférablement en moins de 30 secondes, à partir du moment de l'administration ; et/ou
ii) ladite forme de dosage libère la nicotine dans les 30 minutes suivant l'administration à la cavité orale, préférablement dans les 20 minutes, à partir du moment de l'administration ; et/ou
iii) la réduction de la ou des sensations de perturbations organoleptiques ne détériore pas de manière notable l'effet pharmaceutique de la nicotine et/ou de l'API.

12. Forme de dosage selon l'une quelconque des revendications 1 à 11, **caractérisée en ce qu'**une dose unitaire de celle-ci possède un noyau comprenant
- nicotine en une quantité correspondant à 0,5 mg à 12 mg de nicotine base,
- sorbitol 0 à 99,15 % (p/p),
- xylitol 0 à 99,15 % (p/p),
- mannitol 0 à 99,15 % (p/p),
- bicarbonate de sodium 0 à 1,0 % (p/p),
- carbonate de sodium 0 à 1,0 % (p/p),
- arôme 0,05 à 2,5 % (p/p),
- édulcorant d'intensité élevée ou combinaison d'édulcorants d'intensité élevée 0 à 0,25 % (p/p),
- stéarate de magnésium 0,5 à 2,5 % (p/p).

13. Forme de dosage selon l'une quelconque des revendications 1 à 12, **caractérisée en ce que** le revêtement comprend
i) polymère filmogène environ 80 % (p/p), tensioactif environ 0,3 % (p/p),
édulcorant d'intensité élevée ou combinaison d'édulcorants d'intensité élevée environ 8 % (p/p),
arôme environ 12 % (p/p) ; ou
ii) polymère filmogène 80,5 % (p/p),
plastifiant 8 % (p/p)
tensioactif 0,1 % (p/p),
composant colorant 6 % (p/p)
édulcorant d'intensité élevée ou combinaison d'édulcorants d'intensité élevée environ 4 % (p/p),
arôme 1,25 % (p/p) ; ou
iii) polymère filmogène de 44,5 à 97,0 % (p/p), plastifiant de 0 à 25,0 % (p/p),
tensioactif de 0 à 0,5 % (p/p),
composant colorant de 0 à 10 % (p/p), édulcorant d'intensité élevée ou combinaison d'édulcorants d'intensité élevée de 0,5 à 10 % (p/p),
arôme de 2,5 à 10 % (p/p).

14. Forme de dosage selon l'une quelconque des revendications 1 à 11, **caractérisée en ce qu'**une dose unitaire comprend
i) les quantités ci-dessous en mg respectivement dans le noyau et le revêtement, les quantités respectives pouvant varier dans un intervalle de ± 15 % (p/p), préférablement dans un intervalle de ± 5 % (p/p)
**Noyau**
| | |
|---|---|
| Mannitol | 175,78 |
| Hydrogénocarbonate de potassium | 0,45 |
| Carbonate de sodium | 3,67 |
| Alginate de sodium | 10,30 |
| Gomme xanthane | 1,99 |
| Polycarbophyle de calcium | 5,13 |
| Complexe nicotine résine | 22,22 |
| Hydrogénocarbonate de potassium | 0,58 |
| Carbonate de sodium anhydre | 4,63 |
| Aspartame | |
| Acésulfame potassique | 1,50 |
| Arôme menthe | 21,25 |
| Stéarate de magnésium | 2,50 |
| Poids total du noyau de comprimé en mg | 250 |
**Revêtement**
| | |
|---|---|
| Hydroxypropylméthylcellulose | 4,83 |
| Dioxyde de titane | 0,19 |
| Polyéthylèneglycol 400* | 0,094 |
| Laurylsulfate de sodium | 0,019 |
| Aspartame | 0,31 |
| Acésulfame potassique | 0,19 |
| Arôme menthe | 3,00 |
; ou
ii) ci-dessous en mg respectivement dans le noyau et le revêtement, les quantités respectives pouvant varier dans un intervalle de ± 15 % (p/p), préférablement dans un intervalle de ± 5 % (p/p)
**Noyau**
| | |
|---|---|
| Mannitol | 1 034,85 |
| Hydrogénocarbonate de potassium | 2,80 |
| Carbonate de sodium | 22,75 |
| Alginate de sodium | 63,70 |
| Gomme xanthane | 12,25 |
| Polycarbophyle de calcium | 31,73 |
| Complexe nicotine résine | 22,22 |
| Aspartame | 6,00 |
| Arôme menthe | 1,2 |
| Stéarate de magnésium | 2,50 |
| Poids total du noyau de comprimé en mg | 1 200 |
**Revêtement**
| | |
|---|---|
| Hydroxypropylméthylcellulose | 23,19 |
| Dioxyde de titane | 0,90 |
| Polyéthylèneglycol 400 | 0,45 |
| Laurylsulfate de sodium | 0,090 |
| Aspartame | 1,47 |
| Acésulfame potassique | 0,90 |
| Arôme menthe | 3,00 |

15. Forme de dosage selon l'une quelconque des revendications 1 à 14 pour une utilisation dans :
i) une thérapie, la thérapie étant un traitement d'une maladie choisie dans le groupe constitué par une dépendance au tabac ou à la nicotine, la maladie d'Alzheimer, la maladie de Crohn, la maladie de Parkinson, le syndrome de Gilles de la Tourette, la colite ulcéreuse et un gain de poids après un arrêt de tabagisme, spécifiquement une dépendance au tabac ou à la nicotine ; ou
ii) un procédé de traitement d'une dépendance au tabac ou à la nicotine, de la maladie d'Alzheimer, de la maladie de Crohn, de la maladie de Parkinson, du syndrome de Gilles de la Tourette, d'une colite ulcéreuse et d'un gain de poids après un arrêt de tabagisme, le procédé comprenant l'administration par voie orale de la forme de dosage selon l'une quelconque des revendications 1 à 14 au patient, éventuellement la forme de dosage étant retenue dans la cavité buccale suivant l'administration au patient de sorte que la nicotine soit absorbée par la muqueuse de la cavité buccale et/ou par la muqueuse du pharynx.

16. Système thérapeutique comprenant une forme de dosage selon l'une quelconque des revendications 1 à 15 comprenant de la nicotine sous une forme quelconque.
